# EUROPEAN PATENT APPLICATION

(11) **EP 1 302 212 A2**
(43) Date of publication of application: **16.04.2003**
(21) Application number: 02022652.8
(22) Date of filing: 09.10.2002
(51) Int. Cl.: A61M 16/06

(54) **Forehead support for a facial mask**

(30) Priority: 10.10.2001 NZ 51475001; 12.06.2002 NZ 51954102
(71) Applicant: Fisher & Paykel Healthcare Limited, East Tamaki, Auckland (NZ)
(72) Inventor: Gradon, Lewis George, Howick, Auckland (NZ); Nightingale, Chris Earl, St. Heliers, Auckland (NZ); McAuley, Alastair Edwin, Remuera, Auckland (NZ); Milivojevic, Ivan, Takapuna, Auckland (NZ)
(74) Representative: Brown, John David

(57) **Abstract**

A device for delivering a supply of gases to a user comprising: a patient interface, in use in fluid communication with said supply of gases, a forehead support adapted to pivot about said interface and in use rest on the forehead of a user and configurable at least in an adjustment mode for selecting a desired fixed relation and a secured mode for securing said desired fixed relation. In one embodiment of the patient interface a mask seals against the face of said user and deliver said gases to the nasal cavity and/or oral cavity and/or throat of said user without substantial pressure on the users face.

## Description

### FIELD OF INVENTION

This invention relates to patient interfaces particularly though not solely for use in delivering CPAP therapy to patients suffering from obstructive sleep apnoea (OSA).

### BACKGROUND OF THE INVENTION

In the art of respiration devices, there are well known variety of respiratory masks which cover the nose and/or mouth of a human user in order to provide a continuous seal around the nasal and/or oral areas of the face such that gas may be provided at positive pressure within the mask for consumption by the user. The uses for such masks range from high altitude breathing (i.e. aviation applications) to mining and fire fighting applications, to various medical diagnostic and therapeutic applications.

One requisite of such respiratory masks has been that they provide as effective seal against the user's face to prevent leakage of the gas being supplied. Commonly, in prior mask configurations, a good mask-to-face seal has been attained in many instances only with considerable discomfort for the user. This problem is most crucial in those applications, especially medical applications, which require the user to wear such a mask continuously for hours or perhaps even days. In such situations, the user will not tolerate the mask for long durations and optimum therapeutic or diagnostic objectives thus will not be achieved, or will be achieved with great difficulty and considerable user discomfort.

US Patent No. 5,243,971 and US Patent No. 6,112,746 are examples of prior art attempts to improve the mask system. US Patent No. 5,570,689 and PCT publication No. WO 00/78384, and US Patent No. 6,119,693 are examples of attempts to improve the forehead rest.

### SUMMARY OF THE INVENTION

It is an object of the present invention to attempt to provide a patient interface which goes some way to overcoming the abovementioned disadvantages in the prior art or which will at least provide the industry with a useful choice.

Accordingly in one aspect the present invention consists in a patient interface, in use in fluid communication with said supply of gases,
a forehead support adapted to pivot about said interface and in use rest on the forehead of a user and means for securing said forehead support in a substantially fixed relation to said interface and configurable at least in an adjustment mode for selecting a desired fixed relation and a secured mode for securing said desired fixed relation.

Preferably said interface is a mask and said mask and said support being configured to in use substantially seal against the face of said user without substantial pressure there to and deliver said gases to the nasal cavity and/or oral cavity and/or throat of said user.

Preferably said desired fixed relation lies at any point within a predetermined angular range.

Preferably said means for securing further comprises a friction engagement whereby said bridge member may be selectively engaged to said nasal mask in said desired fixed relation by a friction engagement.

Preferably said friction engagement comprises an adjustable clamp engaging at least a first flange of said nasal mask with a second flange on said bridge member in a fixed position relative thereto.

Preferably said clamp comprises a elongate member extending through apertures in at least said first flange and said second flange whereby the rotation thereof, through a helical thread acting on either said first flange, said second flange, said bridge member and/or said interface, thereby increasing or decreasing the compressive pressure of said first flange on said second flange and thereby varying the frictional engagement there between.

Preferably said first flange said second flange are flat

Preferably said first flange and said second flange are conical.

In a second aspect a CPAP system for delivering gases to a user
characterised in that
a user interface communicates said gases to said user according to the device claimed in any of the proceeding claims.

Preferably said CPAP system is further characterised in that a humidifier in fluid communication with said interface variably humidifiers said gases prior to delivering to said patient.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

The invention consists in the foregoing and also envisages constructions of which the following gives examples.

### BRIEF DESCRIPTION OF THE DRAWINGS

One preferred form of the present invention will now be described with reference to the accompanying drawings in which;
Figure 1 is a block diagram of a humidified continuous positive airway pressure (system) as might be used in conjunction with the present invention,
Figure 2 is an illustration of the nasal mask in use according to the preferred embodiment of the present invention,
Figure 3 shows a perspective view of the mask with cushion,
Figure 4 is a cutaway view of the mask showing the cushion,
Figure 5 is a cutaway view of the periphery of the outer membrane,
Figure 6 is a cutaway view of the periphery of the mask body portion,
Figure 7 shows the forehead rest in isolation,
Figure 8 shows the pivoting forehead rest with a locking mechanism,
Figure 9 is a perspective view of the mask showing the friction engagement,
Figure 10 is an alternate perspoctive view of the mask showing the friction engagement, and
Figure 11 is a cross section view of the friction engagement.

### DETAILED DESCRIPTION

The present invention provides improvements in the delivery of CPAP therapy. In particular a patient interface is described which is quieter for the user to wear and reduces the side leakage as compared with the prior art It will be appreciated that the patient interface as described in the preferred embodiment of the present invention can be used in respiratory care generally or with a ventilator but will now be described below with reference to use in a humidified CPAP system. It will also be appreciated that the present invention can be applied to any form of patient interface including, but not limited to, nasal masks, oral masks and mouthpieces.

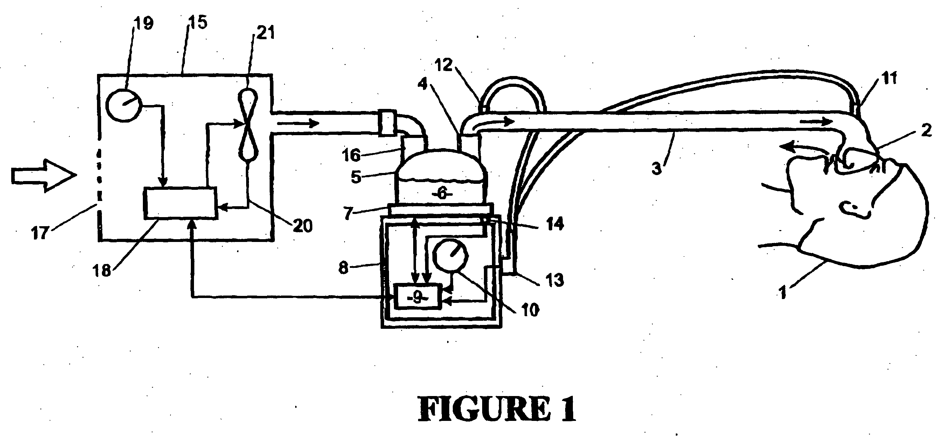

With reference to Figure 1 a humidified Continuous Positive Airway Pressure (CPAP) system is shown in which a patient 1 is receiving humidified and pressurised gases through a patient interface 2 connected to a humidified gases transportation pathway or inspiratory conduit 3. It should be understood that delivery systems could also be VPAP (Variable Positive Airway Pressure) and BiPAP (Bi-level Positive Airway Pressure) or numerous other forms of respiratory therapy. Inspiratory conduit 3 is connected to the outlet 4 of a humidification chamber 5 which contains a volume of water 6. Inspiratory conduit 3 may contain heating means or heater wires (not shown) which heat the walls of the conduit to reduce condensation of humidified gases within the conduit. Humidification chamber 6 is preferably formed from a plastics material and may have a highly heat conductive base (for example an aluminium base) which is in direct contact with a heater plate 7 of humidifier 8. Humidifier 8 is provided with control means or electronic controller 9 which may comprise a microprocessor based controller executing computer software commands stored in associated memory.

Controller 9 receives input from sources such as user input means or dial 10 through which a user of the device may, for example, set a predetermined required value (preset value) of humidity or temperature of the gases supplied to patient 1. The controller may also receive input from other sources, for example temperature and/or flow velocity sensors 11 and 12 through connector 13 and heater plate temperature sensor 14. In response to the user set humidity or temperature value input via dial 10 and the other inputs, controller 9 determines when (or to what level) to energise heater plate 7 to heat the water 6 within humidification chamber 5. As the volume of water 6 within humidification chamber 5 is heated, water vapour begins to fill the volume of the chamber above the water's surface and is passed out of the humidification chamber 5 outlet 4 with the flow of gases (for example air) provided from a gases supply means or blower 15 which enters the chamber through inlet 16. Exhaled gases from the patient's mouth are passed directly to ambient surroundings in Figure 1.

Blower 15 is provided with variable pressure regulating means or variable speed fan 21 which draws air or other gases through blower inlet 17. The speed of variable speed fan 21 is controlled by electronic controller 18 (or alternatively the function of controller 18 could carried out by controller 9) in response to inputs from controller 9 and a user set predetermined required value (preset value) of pressure or fan speed via dial 19.

### Mask

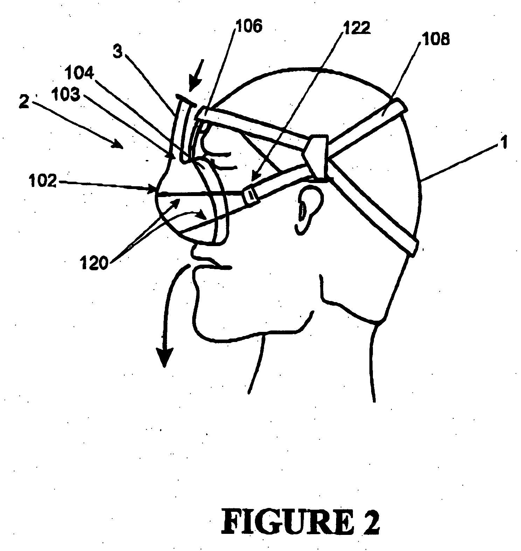

According to a first embodiment of the present invention the patient interface is shown in Figure 2 as a mask This may be also a nasal mask, a full face mask, a oral mask endotracheal tube, nasal cannula or other breathing assistance interface as are known in the art. The mask includes a hollow body 102 with an inlet 103 connected to the inspiratory conduit 3. The mask 2 is positioned around the nose of the user 1 with the headgear 108 secured around the back of the head of the patient 1. The restraining force from the headgear 108 on the hollow body 102 and the forehead rest 106 ensures enough compressive force on the mask cushion 104, to provide an effective seal against the patient's face.

The hollow body 102 is constructed of a relatively inflexible material for example, polycarbonate plastic. Such a material would provide the requisite rigidity as well as being transparent and a relatively good insulator. The expiratory gases can be expelled through a valve (not shown) in the mask, a simple vent (not shown), a further expiratory conduit (not shown), or any other such method as is known in the art.

### Mask Cushion

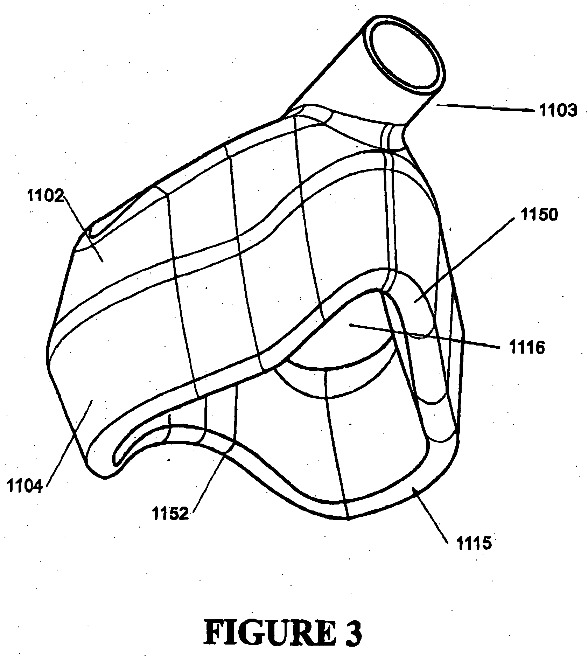

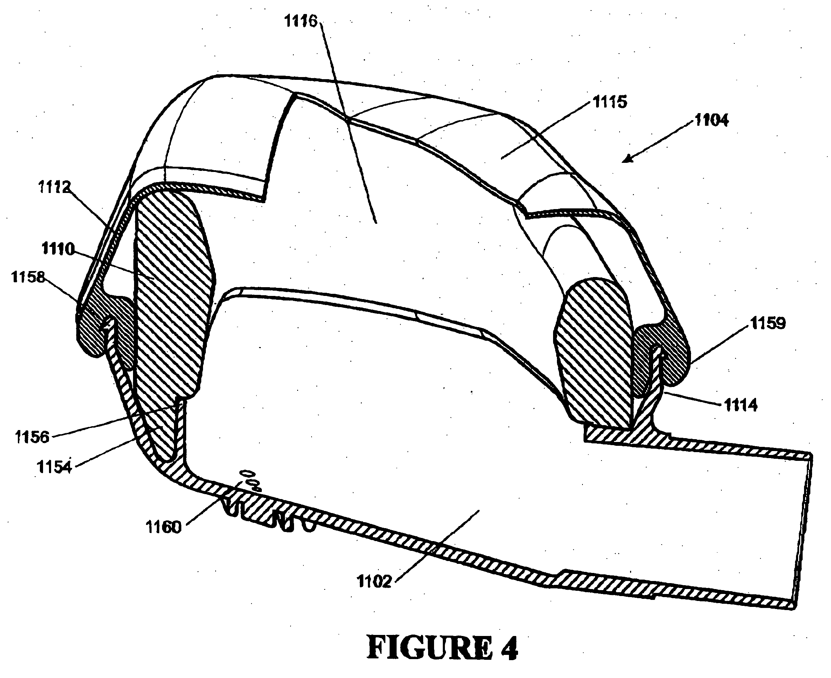

Referring now to Figures 3 and 4 in particular, the mask cushion 1104 is provided around the periphery of the nasal mask 1102 to provide an effective seal onto the face of the user to prevent leakage. The mask cushion 1104 is shaped to approximately follow the contours of a patient's face. The mask cushion 104 will deform when pressure is applied by the headgear 1108 to adapt to the individual contours of any particular user. In particular, there is an indented section 1150 intended to fit over the bridge of the user's nose as well as a less indented section 1152 to seal around the section beneath the nose and above the upper lip.

In Figure 4 we see that the mask cushion 1104 is composed of a inner foam cushion 1110 covered by an outer sealing sheath 1112. The inner cushion 1110 is constructed of a resilient material for example polyurethane foam, to distribute the pressure evenly along the seal around the user's face. The inner cushion 1110 is located around the outer periphery 1114 of the open face 1116 of the hollow body 1102. Similarly the outer sheath 1112 may be commonly attached at its base 1113 to the periphery 1114 and loosely covers over the top of the inner cushion 1110.

In the preferred embodiment shown in Figures 3-6 the bottom of the inner cushion 1110 fits into a generally triangular cavity 1154 in the hollow body 1102. The cavity 1154 is formed from a flange 1156 running mid-way around the interior of the hollow body.

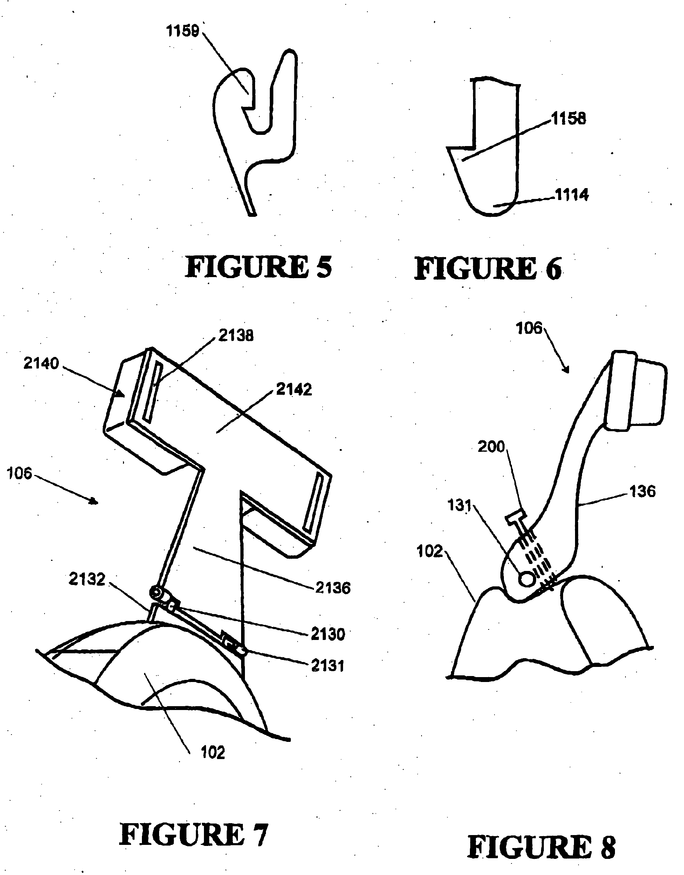

The outer sheath 1112 fits in place over the cushion 1110, holding it in place. The sheath 1112 is secured by a snap-fit to the periphery 1114 of the hollow body. In Figures 5-6 the periphery 1114 is shown including an outer bead 1158. The sheath 1112 includes a matching bead 1159, whereby once stretched around the periphery, the two beads engage to hold the sheath in place. However it will be appreciated the invention is not limited to this form of sealing.

### Forehead Rest

In the preferred embodiment of the present invention the nasal mask 2102 includes a pivoting forehead rest 106 (seen in Figures 2 and 7). The attachment of the forehead rest 106 to the hollow body 102 effectively allows the forehead rest 106 to pivot towards and away from the user but with no lateral movement

In one form shown in Figure 7, pins 2130 are provided mounted on a base 2132 attached to the hollow body 102. These pins 2130 are co-axial within cylinders 2131 mounted on a bridge member 2136.

At the top end 2142 (around the user's forehead) of the bridge member 2136 harnessing slots 2138 are provided which allow straps from the headgear to be inserted to secure the mask to the headgear. For the user's comfort one or more resilient cushions 2140 are provided underneath the top end 2142 of the bridge member 2136, which rest on the forehead of the user. The cushion 2140 might be constructed of silicon or any foam materials as is known in the art for providing cushioning.

In a further embodiment the forehead rest 106 described previously may include a weakened section 2130 at its base 2132 which allows the joining member 2136 to pivot from the hollow body 102. The bridge member extends up to the forehead of the user. In a further alternative the mask may include a vertical upwardly extending inlet. In this case the member 2136 is hinged at its base 2132 to either side of the inlet passage. Again the member would then extend to the forehead.

In a still further embodiment shown in Figure 8 the forehead rest 106 is shown with the pivoting action which can be locked in a single position. As before the bridge member 136 pivots by virtue of pins from the hollow body 102 co-operating with cylinders 131 on the bridge member 136. The locking action is provided by an engaging clip 200 which is attached through an aperture at the base of the bridge member 136. The lock and clip 200 has a number of ribs which engage with at least two ribs on the interior of the aperture which allows it to lock and place it in at least an upper position and a lower position. In the upper position the locking clip 200 is clear of the hallow body 102 and allows the bridge member 136 to pivot freely. In the lower position the locking pin 200 engages with a cavity on the surface of the said hollow body 102 which locks said bridge member 136 at a predetermined angular position with respect to said hollow body 102. In the preferred embodiment this position provides the maximum offset of the upper section of the mask from the bridge of the patients nose.

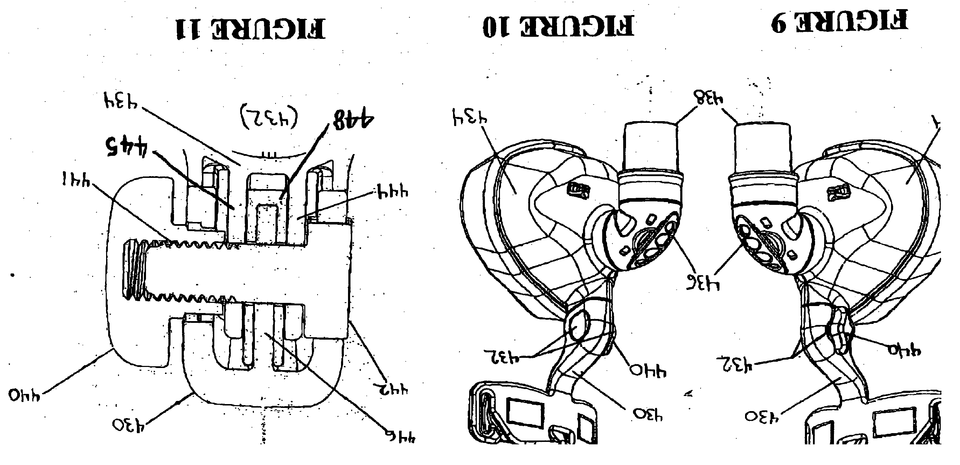

In a still further embodiment shown in Figures 9 to 11 the bridge member 430 according to the present invention is shown including a friction engagement 432 with the mask body 434. In this embodiment the mask body 434 is shown with an elbow connector 436 connected to the inlet conduit 438. An adjustment knob 440 provided on the side of the bridge member 432 allows adjustment. With the knob 440 in a loosened position the bridge member 430 may be pivoted to any desired angle with respect to the mask body 434. Once in the desired position the knob 440 may be configured to a tightened position whereby the angle of the bridge member 430 relative to the mask body 434 is substantially fixed by virtue of the friction engagement in the pivot parts. Knob 440 may also be configured in a partially tightened position whereby the pivot angle may be adjusted by applying a large force between the two.

In more detail shown in Figure 11 the knob 440 includes a helical thread engaging with a reciprocal helical thread 441 on pin 442 running transversely through the bridge member 430. The pin also runs through apertures in two flanges 444, 445 extending up from the mask body 434 and an internal flanges 446 from the bridge member 430. In this fashion the body flanges 444, 445 and bridge flange 446 may fictionally engage (optionally also with the inner surfaces of the bridge member 430) once the knob 440 is in a tightened position. More preferably washer 448 is provided between body flanges 444,445 and bridge flange 446. Washer 448 improves the friction engagement and may be formed as a single hard rubber insert, or two separate washers. Adjustment may either be allowed in a finite number of predetermined positions or more preferably may be completely variable.

In a further variation the body flange and bridge flange may be conical, or any other configuration allowing pivoting as well as engagement in a desired relation.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A device for delivering a supply of gases to a user comprising:
a patient interface, in use in fluid communication with said supply of gases,
a forehead support adapted to pivot about said interface and in use rest on the forehead of a user and means for securing said forehead support in a substantially fixed relation to said interface and configurable at least in an adjustment mode for selecting a desired fixed relation and a secured mode for securing said desired fixed relation.

2. A device as claimed in claim 1 wherein said interface is a mask and said mask and said support being configured to in use substantially seal against the face of said user without substantial pressure there to and deliver said gases to the nasal cavity and/or oral cavity and/or throat of said user.

3. A device as claimed in claims 1 or 2 wherein said desired fixed relation lies at any point within a predetermined angular range.

4. A device as claimed in claim 3 wherein said means for securing further comprises a friction engagement whereby said bridge member may be selectively engaged to said nasal mask in said desired fixed relation by a friction engagement.

5. A device as claimed in claim 4 wherein said friction engagement comprises an adjustable clamp engaging at least a first flange of said nasal mask with a second flange on said bridge member in a fixed position relative thereto.

6. A device as claimed in claim 5 wherein said clamp comprises a elongate member extending through apertures in at least said first flange and said second flange whereby the rotation thereof, through a helical thread acting on either said first flange, said second flange, said bridge member and/or said interface, thereby increasing or decreasing the compressive pressure of said first flange on said second flange and thereby varying the frictional engagement there between.

7. A device as claimed in claim 16 wherein said first flange said second flange are flat.

8. A device as claimed in claim 16 wherein said first flange and said second flange are conical.

9. A CPAP system for delivering gases to a user
**characterised in that**
a user interface communicates said gases to said user according to the device claimed in any of the proceeding claims.

10. A CPAP system as claimed in claim 9 further **characterised in that** a humidifier in fluid communication with said interface variably humidifiers said gases prior to delivering to said patient.
